# EUROPÄISCHE PATENTSCHRIFT

(11) **EP 2 814 550 B1**
(45) Veröffentlichungstag und Bekanntmachung des Hinweises auf die Patenterteilung: **30.03.2016**
(21) Anmeldenummer: 13702429.5
(22) Anmeldetag: 29.01.2013
(51) Int. Cl.: A61M 16/00

(54) **BEATMUNGSSYSTEM**
RESPIRATION SYSTEM
SYSTÈME DE RESPIRATION

(30) Priorität: 16.02.2012 DE 102012003115
(43) Veröffentlichungstag der Anmeldung: 24.12.2014
(73) Patentinhaber: Drägerwerk AG & Co. KGaA, 23558 Lübeck (DE)
(72) Erfinder: MERSMANN, Stefan, 23564 Lübeck (DE); KATSCHEWITZ, Thomas, 23562 Lübeck (DE); NEUMANN, Andreas, 23628 Klempau (DE); JOLLIET, Philippe, 1011 Lausanne (CH)
(74) Vertreter: Guthöhrlein, Gerhard
(86) Internationale Anmeldenummer: PCT/EP2013/051666
(87) Internationale Veröffentlichungsnummer: WO 2013/120690

(56) Entgegenhaltungen:
- EP-A1- 2 368 593
- WO-A1-97/20592

## Beschreibung

Die vorliegende Erfindung betrifft ein Beatmungssystem zur nicht-invasiven Überdruckbeatmung mit einer Druckquelle zur Bereitstellung von Atemgas mit steuerbarem Druck, die von einer Steuer- und Auswerteeinheit gesteuert wird, und mit der Steuer- und Auswerteeinheit verbundenen Sensoren, die die Erfassung des Leckagevolumens, der Spontanatemfrequenz, des Tidalvolumens und der Inspirationszeit ermöglichen.

Nicht-invasive Ventilation (NIV) bezeichnet eine maschinelle Atemhilfe beziehungsweise Beatmungsform, bei der der Atemwegszugang zum Patienten nicht über Endotracheal- bzw. Tracheostomietubus (invasiv) erfolgt, sondern über eine dicht sitzende Nasen- oder Gesichtsmaske (nicht-invasiv). In der nicht-invasiven Beatmungstherapie wird zwischen Unterdruckbeatmung (NPV - Negative Pressure Ventilation) und Überdruckbeatmung (NIPPV - Non-invasive Positive Pressure Ventilation) mit den Beatmungsmustern BIPAP (Biphasic Positive Airway Pressure) sowie CPAP/ASB (Continuous Positive Airway Pressure / Assisted Spontaneous Breathing) unterschieden. Die vorliegende Erfindung beschäftigt sich mit der NIPPV in CPAP/ASB.

Eine beispielhafte NIV-Vorrichtung ist aus dem Dokument WO 97/20592 bekannt.

Nicht-invasive Ventilation wird zur Therapie vielfältiger Formen der respiratorischen Insuffizienz eingesetzt und hat sich als Ergänzung zur konventionellen, d.h. invasiven, Entwöhnung vom Beatmungsgerät durchgesetzt. So wird die Beatmungsform CPAP/ASB mit NIV für die Regulierung von Oxigenations- und Ventilationsstörungen verwendet.

Der Einsatz von NIV wird insbesondere dann empfohlen, wenn Patienten nach einer vorangehenden konventionellen Entwöhnung extubiert werden und dennoch eine signifikante hyperkapnische, respiratorische Insuffizienz zeigen. In diesen Fällen konnte mit NIV sowohl die Beatmungsdauer reduziert als auch die die Mortalität steigernde Rate von Re-Intubationen verringert werden.

Allgemeine Vorteile der NIV-Beatmung sind:
- Patient toleriert die Beatmung besser.
- Es ist keine oder nur eine geringe Sedierung erforderlich.
- Der Patient kann kommunizieren/mit dem Patienten kann kommuniziert werden.
- Eine orale Ernährung ist möglich.
- Die Atemluft wird physiologisch adäquat befeuchtet.
- Der Patient kann besser mobilisiert werden (z.B. im Sitzen).

Vorteile von NIV gegenüber invasiver Beatmung:
- Kürzere Beatmungsdauer,
- Kürzerer Aufenthalt auf der Intensivstation,
- Weniger nosokomielle Infektionen, insbesondere Pneumonien, insbesondere durch Re-intubationen,
- Geringere Mortalität,
- Geringe Therapiekosten.

Voraussetzungen und Indikationen für NIV:
- Der Patient muss wach und kooperativ sein,
- Atemantrieb sowie Schluck- und Schutzreflexe müssen vorhanden,
- Hämodynamische Stabilität,
- Pulmonale Indikationen:
   - Chronisch respiratorische Insuffizienz.
      - COPD
      - Obstruktive Schlafapnoe
      - Atemantriebsstörungen
      - Neuromuskuläre Störungen
   - Akut respiratorische Insuffizienz
      - Akute COPD
      - Pneunomie
      - Lungenembolie
      - Entwöhnung
- Kardiale Indikationen
   - Kardiogenes Lungenödem.

Die für die Respiratoren Dräger EvitaXL und Dräger Evita Infinity V500 verfügbare Beatmungsoption SmartCare/PS bietet eine automatisierte Entwöhnungsstrategie für die invasive Beatmungsform CPAP/ASB. Hierbei wird gemäß einer klinischen Leitlinie die inspiratorische Druckunterstützung (ASB) am Respirator sukzessive und in kurzen Zeitabständen zu minimieren versucht, um schließlich den Patienten autonom über einen 1 bis 2-stündigen Spontanatemversuch bis hin zur Extubationsfähigkeit zu geleiten. Alle 5 Sekunden werden dem wissensbasierten System Messdaten vom Respirator zugeführt (spontane Atemfrequenz, Atemzugvolumen, endtidales Kohlendioxid), mit denen automatisch alle 2 bis 5 Minuten eine mit der klinischen Leitlinie konforme und dem Patientenzustand adäquate Anpassung der Beatmungstherapie vorgenommen wird. In einer multizentrisch randomisierten Studie konnte gezeigt werden, dass mit dieser automatischen Beatmungsanpassung die Beatmungsdauer um 33% und die Entwöhnungsdauer um bis zu 50% reduzierbar ist.

Das oben beschriebene Anpassungsverfahrens SmartCare/PS lässt sich für die nicht-invasive Beatmung mit dem Ziel der postextubativen respiratorischen Stabilisierung nicht unmittelbar anwenden. Wesentliche Gründe hierfür sind:
- Anders als bei SmartCare/PS ist eine aktive Entwöhnung bei NIV nicht das therapeutische Ziel.
- Ein aktiver Spontanatemversuch ist in der NIV nicht erwünscht, im Gegensatz zu SmartCare/PS.
- Die CO₂-Messung unter NIV ist fragil und daher nur bedingt nutzbar, wohingegen der endtidale CO₂-Wert für SmartCare/PS ein wesentlicher Eingangsparameter ist.
- Neben dem ASB müssen weitere Beatmungsparameter verstellt werden.

Insbesondere können mit dem bekannten Verfahren Patienten-Geräte-Asynchronien nicht gezielt vermieden werden. Leckagen, die gerade unter NIV erheblich sein können, werden bei Smart-Care/PS nicht berücksichtigt. Schließlich ist es bei nichtinvasiver Beatmung erforderlich, die Einstellungen häufiger zu kontrollieren.

Es ist Aufgabe der vorliegenden Erfindung, ein Beatmungssystem für die nicht-invasive Beatmung mit dem Beatmungsmuster CPAP/ASB so auszugestalten, dass eine post-extubative respiratorische Stabilisierung des Patienten automatisch vorgenommen wird.

Zur Lösung dieser Aufgabe dient das Beatmungssystem mit den Merkmalen des Patentanspruchs 1.

Erfindungsgemäß ist vorgesehen, dass eine Steuer- und Auswerteeinheit des Beatmungssystems dazu eingerichtet ist, Eingangsgrößen in einer bestimmten Reihenfolge auf das Erfüllen von bestimmten Kriterien zu untersuchen und je nach Ergebnis der Untersuchung gegebenenfalls eine Anpassung der Beatmungsparameter vorzunehmen. Im Zusammenhang mit der vorliegenden Erfindung hat sich herausgestellt, dass als Eingangsgrößen sukzessive folgende Parameter untersucht werden sollten:
- Leckagevolumen (MV_LEAK)
- Spontanatemfrequenz (F_SPON)
- Tidalvolumen (VT)
- Inspirationszeit (TI_SPON).

Als Ausgangsgrößen werden die inspiratorische Druckunterstützung (P_ASB) und die Zeitperiode, in der die inspiratorische Druckunterstützung durchgeführt wird, eingestellt. Es hat sich herausgestellt, dass gerade die im Patentanspruch 1 definierte Reihenfolge und die Überprüfung der angegebenen Kriterien zu einer stabilen automatisierten Führung des Beatmungssystems führen.

Demgemäß muss zunächst das Leckagevolumen daraufhin überprüft werden, ob es oberhalb eines vorgegebenen Grenzwertes liegt. Wenn das der Fall ist, wird die Spontanatemfrequenz daraufhin überprüft, ob sie innerhalb eines vorgegebenen Spontanatemfrequenzintervalls liegt, und das Tidalvolumen daraufhin überprüft, ob es innerhalb eines vorgegebenen Tidalvolumenintervalls liegt, und wenn eine der beiden vorgenannten Bedingungen nicht erfüllt ist, wird eine Alarmmeldung gegeben und zum Ausgangspunkt zurückgekehrt. Andernfalls, wenn das Leckagevolumen oberhalb des vorgegebenen Leckagevolumengrenzwertes liegt und die Spontanatemfrequenz innerhalb des vorgegebenen Spontanatemfrequenzintervalls und das Tidalvolumen innerhalb des vorgegebenen Tidalvolumenintervalls liegt, wird die inspiratorische Druckunterstützung, d.h. der Druck der durch den Atemantrieb bereitgestellt wird, reduziert, bis das resultierende Leckagevolumen unterhalb des vorgegebenen Grenzwertes liegt.

Wenn das gelungen ist, wird geprüft, ob die Spontanatemfrequenz oberhalb eines vorgegeben Maximalwerts oder unterhalb eines vorgegeben Minimalwerts liegt, und wenn ja ein Alarm ausgelöst und zu dem vorhergehenden Schritt zurückgekehrt. Anschließend wird die Spontanatemfrequenz mit dem vorgegebenen Spontanatemfrequenzintervall verglichen und festgestellt, ob die Spontanatemfrequenz unter, im oder über dem vorgegebenen Spontanatemfrequenzintervall liegt. Falls die Spontanatemfrequenz über dem vorgegebenen Spontanatemfrequenzintervall liegt, wird die inspiratorische Druckunterstützung reduziert. Falls die Spontanatemfrequenz unter dem vorgegebenen Intervall liegt, wird die inspiratorische Druckunterstützung erhöht. Wenn hier die inspiratorische Druckunterstützung erhöht oder erniedrigt worden ist, wird zu dem ersten Schritt mit der Überprüfung des Leckagevolumens zurückgekehrt. Nur wenn die Spontanatemfrequenz im vorgegebenen Spontanatemfrequenzintervall liegt, wird die inspiratorische Druckunterstützung unverändert gelassen und zu dem nächsten Prüfschnitt übergegangen.

Als nächstes wird das Tidalvolumen daraufhin überprüft, ob es unter, innerhalb oder über dem vorgegebenen Tidalvolumenintervall liegt. Falls das Tidalvolumen über dem vorgegebenen Tidalvolumenintervall liegt, wird die inspiratorische Druckunterstützung reduziert. Falls das Tidalvolumen unter dem vorgegebenen Tidalvolumenintervall liegt, wird die inspiratorische Druckunterstützung erhöht. Wenn hier die inspiratorische Druckunterstützung erhöht oder erniedrigt worden ist, wird zu dem ersten Schritt mit der Überprüfung des Leckagevolumens zurückgekehrt. Nur im Fall, dass das aktuelle Tidalvolumen im vorgegebenen Tidalvolumenintervall liegt, wird die inspiratorische Druckunterstützung unverändert gelassen und zum nächsten Prüfschritt übergegangen.

Schließlich wird die Zeitspanne oder Zeitperiode, innerhalb der die inspiratorische Druckunterstützung während eines Atemzuges durchgeführt wird, in Abhängigkeit von der Inspirationszeit des Patienten eingestellt. Die Zeitperiode der Druckunterstützung kann zum Beispiel durch ein Kriterium des Parameters % PIF eingestellt werden, wobei PIF für "peak inspiration flow" steht. Eine Zeitperiode von 5% PIF bedeutet dann, dass die inspiratorische Druckunterstützung beendet wird, wenn der Volumenstrom auf 5% gegenüber seinem Spitzenwert in dem jeweiligen Atemzug abgesunken ist. Entsprechend kann auch eine kürzere inspiratorische Druckunterstützung im Verhältnis zur Dauer des Atemzuges gesetzt werden, zum Beispiel 70% PIF, was bedeutet, dass die inspiratorische Druckunterstützung schon beendet wird, wenn der Volumenstrom auf 70% seines Spitzenwertes in diesem Atemzug abgefallen ist.

Die Erfindung wird im Folgenden anhand eines Ausführungsbeispiels in den Zeichnungen beschrieben, in denen
Fig. 1: eine perspektivische Ansicht eines Beatmungssystems zeigt,
Fig. 2: ein Flussdiagramm der Steuerung des erfindungsgemäßen Beatmungssystems zeigt und
Fig. 3: ein Flussdiagramm der Schrittfolgen der Abfragen in einem erfindungsgemäßen Beatmungssystem zeigt.

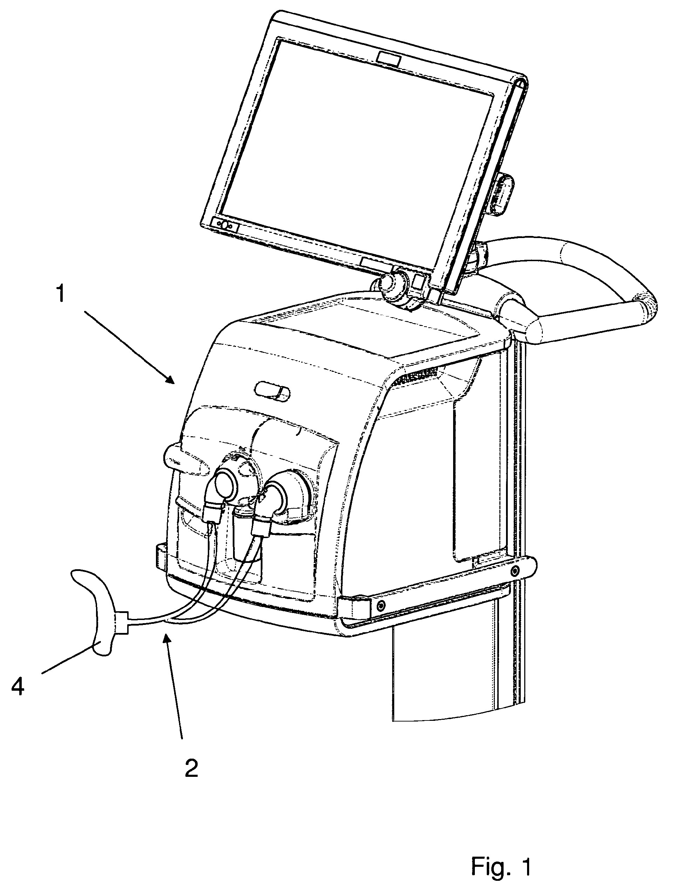

Fig. 1 zeigt ein Beatmungssystem 1 mit einem Schlauchsystem 2, das eine inspiratorische und eine exspiratorische Leitung umfasst, die in ein Y-Stück an einer Beatmungsmaske 4 münden. Innerhalb des Beatmungssystems ist eine Druckquelle und eine Steuer- und Auswerteeinheit untergebracht, die die Ausgabe von Atemgas mit dem gewünschten Druckverlauf aus der Druckquelle steuert. Ferner sind Sensoren zur Erfassung von pneumatischen Größen in üblicher Weise vorhanden, die in der Fig. 1 nicht im einzelnen dargestellt sind. Von den Sensoren werden das Leckagevolumen, die Spontanatemfrequenz, das Tidalvolumen und weitere Parameter in üblicher Weise erfasst.

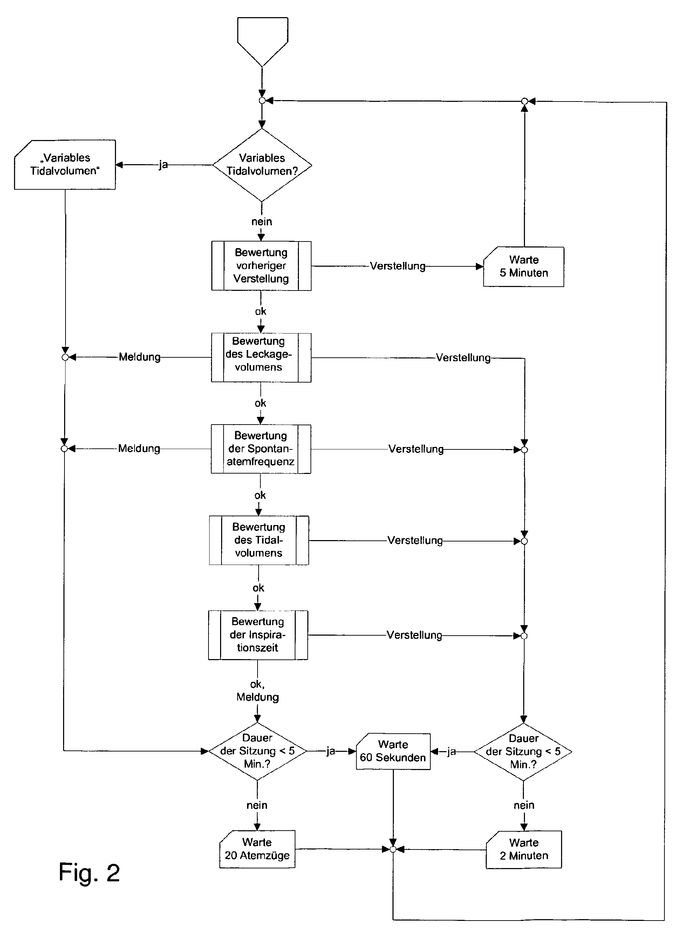

Mit Bezug auf Fig. 2 wird von der Steuer- und Auswerteeinheit zunächst überprüft, ob das Tidalvolumen variabel ist. Wenn nicht, wird die vorherige Verstellung bewertet. Falls die inspiratorische Druckunterstützung zuvor erhöht wurde, wird überprüft, ob hierdurch die Spontanatemfrequenz des Patienten reduziert werden konnte. Wenn das der Fall ist, wird zum nächsten Prüfschritt übergegangen.

Wenn durch die vorherige Erhöhung der inspiratorischen Druckunterstützung weder die Spontanatemfrequenz des Patienten reduziert werden konnte noch das Tidalvolumen sich erhöht hat, wird die vorherige Erhöhung der inspiratorischen Druckunterstützung zurückgenommen und 5 Minuten bis zum nächsten Zyklus gewartet.

Wenn durch die vorherige Erhöhung der inspiratorischen Druckunterstützung zwar nicht die Spontanatemfrequenz des Patienten reduziert werden konnte, sich aber das Tidalvolumen erhöht hat, wird zum nächsten Prüfschritt übergegangen, falls das Tidalvolumen nicht über dem vorgegebenen Tidalvolumenintervall liegt. Sollte das Tidalvolumen über dem vorgegebenen Tidalvolumenintervall liegen, werden zunächst keine weiteren Verstellungen vorgenommen und 5 Minuten bis zum nächsten Zyklus gewartet.

Zu Beginn der automatisierten Einstellung der Beatmungssystems gibt der Benutzer die Systeminformationen zum Patienten: Körpergröße sowie Intervalle, in denen sich das Leckagevolumen, die Spontanatemfrequenz und das Tidalvolumen bewegen dürfen. Innerhalb der ersten 5 Minuten nach Start des Beatmungssystems wird alle 60 Sekunden eine Bewertung aus den Eingangswerten P_ASB, PIF, VT, F_SPON, TI_SPON und MV_LEAK ermittelt, die zu einer autonomen Verstellung der Einstellgrößen P_ASB und/oder PIF führen kann. Nach den ersten 5 Minuten wird alle 20 Atemzüge die Bewertung wiederholt und/oder eine Verstellung vorgenommen, wobei nach einer autonomen Verstellung jeweils 2 Minuten gewartet wird.

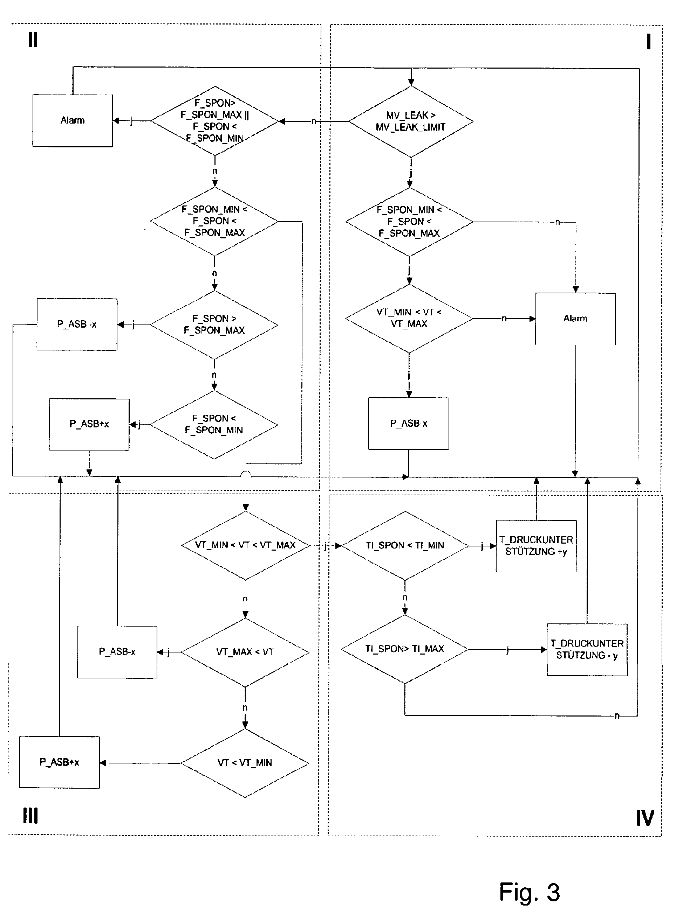

Der übergeordnete, zyklisch sich wiederholende Prüfablauf stellt sich wie folgt dar: Nach der Prüfung des aktuellen Atemzugvolumens auf Bereichsüberschreitung und retrospektive Auswertung der vergangenen autonomen Parameterverstellung (Assess prior adjustment) und entsprechender Korrektur, wird basierend auf einer fest vorgegebenen Auswertereihenfolge die Stabilisierung automatisch in vier Schritten angegangen, wobei diese Schrittfolgen in dem Flussdiagramm in Fig. 3 visualisiert ist, wobei die mit I Bis IV bezeichneten Blöcken den folgenden Schritten 1. bis 4. bzw. den Schritten i) bis iv) in Anspruch 1 entsprechen:
1. Analysieren des vorhandenen Leckagevolumens MV_LEAK und, wenn das Leckagevolumen einen Grenzwert überschreitet, Prüfung, ob die Spontanatemfrequenz F_SPON innerhalb eines vorgegebenen Spontanatemfrequenzintervalls und ob das Tidalvolumen VT innerhalb eines vorgegebenen Tidalvolumenintervalls liegt. Wenn eine oder beide der letzten beiden Bedingungen nicht erfüllt ist, wird ein Alarm ausgelöst und zum Ausgangspunkt zurückgekehrt. Andernfalls, und wenn das Leckagevolumen oberhalb des Grenzwertes liegt, wird die inspiratorische Druckunterstützung P_ASB reduziert (was in Fig. 3 durch P_ASB-x angedeutet ist), um das Leckagevolumen zu reduzieren, und zum Ausgangspunkt zurückgekehrt; wenn das Leckagevolumen unter dem Grenzwert liegt, wird mit dem folgenden Schritt 2. fortgefahren (dieser Schritt ist in Fig. 2 mit "Bewertung des Leckagevolumens" bezeichnet);
2. wenn die Spontanatemfrequenz F_SPON oberhalb eines vorgegeben Maximalwerts oder unterhalb eines vorgegeben Minimalwerts liegt, wird ein Alarm ausgelöst und zu Schritt 1. zurückgekehrt; andernfalls wird die Spontanatemfrequenz F_SPON mit dem vorgegebenen Spontanatemfrequenzintervall, das innerhalb des Intervalls zwischen Maximalwert und Minimalwert liegt, verglichen, und falls die Spontanatemfrequenz F_SPON oberhalb des vorgegeben Spontanatemfrequenzintervalls liegt, die inspiratorische Druckunterschützung reduziert (angedeutet durch P_ASB-x), und falls die Spontanatemfrequenz unter dem vorgegebenen Intervall liegt, die inspiratorische Druckunterschützung erhöht(P_ASB+x); nach einer Erhöhung oder Reduzierung der inspiratorischen Druckunterschützung wird zu Schritt 1. zurückgekehrt, und falls die Spontanatemfrequenz F_SPON im vorgegebenen Spontanatemfrequenzintervall liegt, wird die inspiratorische Druckunterschützung unverändert belassen und zu Schritt 3. übergegangen (dieser Schritt ist in Fig. 2 mit "Bewertung der Spontanatemfrequenz" bezeichnet);
3. in Abhängigkeit vom Tidalvolumen VT wird die inspiratorische Druckunterstützung P_ASB verstellt oder nicht, nämlich P_ASB reduziert (P_ASB-x), wenn das Tidalvolumen VT oberhalb des vorgegebenen Tidalvolumenintervalls liegt, oder P_ASB erhöht (P_ASB+x), wenn das Tidalvolumen VT unter dem vorgegebenen Tidalvolumenintervall liegt. Nach einer so ausgelösten Erhöhung oder Reduzierung wird wieder zum Schritt 1. zurückgekehrt. Nur wenn das Tidalvolumen VT im vorgegebenen Tidalvolumenintervall liegt, wird P_ASB unverändert gelassen und zu Schritt 4. übergegangen, (dieser Schritt ist in Fig. 2 mit "Bewertung des Tidalvolumens" bezeichnet);
4. die Zeitperiode, in der die inspiratorische Druckunterstützung während eines Atemzuges durchgeführt wird, wird in Abhängigkeit von der Inspirationszeit eingestellt, indem die Zeitperiode der inspiratorischen Druckunterstützung erhöht wird (T_DRUCKUNTERSTÜTZUNG+y), wenn die Inspirationszeit TI_SPON unter einem vorgegebenen Inspirationszeitintervall liegt, und indem sie verringert wird (T_DRUCKUNTERSTÜTZUNG-y), wenn die Inspirationszeit über dem vorgegebenen Inspirationsintervall liegt, wobei sie unverändert belassen wird, wenn die Inspirationszeit im vorgegebenen Inspirationszeitintervall liegt, und zu Schritt 1. zurückkehrt (dieser Schritt ist in Fig. 2 mit "Bewertung der Inspirationszeit" bezeichnet).

Im Folgenden sind Situationen eines Beatmungsvorgangs in einer Tabelle mit Werten für das Leckagevolumen, die Spontanatemfrequenz, das Tidalvolumen und die Inspirationszeit aufgeführt, wobei die jeweilige Schlussfolgerung und die daraus resultierende Änderung von P_ASB und %PIF aufgeführt sind.

In diesem Beispiel beträgt der Grenzwert für das Leckagevolumen 15 l/min. Das vorgegebene Spontanatemfrequenzintervall ist 14 - 29 bpm (Atemzüge pro Minute). Der vorgegebene Maximalwert der Spontanatemfrequenz, bei dessen Überschreitung ein Alarm ausgelöst und zu Schritt 1 zurückgekehrt wird, beträgt 40 bpm. Der vorgegebene Minimalwert, unterhalb dessen ebenfalls ein Alarm ausgelöst wird und zum ersten Schritt zurückgekehrt wird, beträgt 8 bpm.

Das vorgegebene Tidalvolumenintervall ist 5 - 10 ml/kg (Atemvolumen pro kg Körpergewicht).

Das vorgegebene Inspirationszeitintervall ist 0,6 - 1,2 s.

### Bewertung des Leckagevolumens

| **Leckagevolumen [l/min]** | **Diagnose** | **Δ PS [mbar]** | **PIF** |
|---|---|---|---|
| 0 - 15 | Adäquate Unterstützung | keine Verstellung | keine Verstellung |
| 16 - 30 | Leckagen überprüfen | - 1 | keine Verstellung |

### Bewertung der Spontanatemfrequenz

| **Spontanatemfrequenz [bpm]** | **Diagnose** | **Δ PS [mbar]** | **PIF** |
|---|---|---|---|
| > 40 | Tachypnoe | keine Verstellung | keine Verstellung |
| 36 - 40 | Zu niedrige Unterstützung | + 3 | keine Verstellung |
| 33 - 35 | Zu niedrige Unterstützung | + 2 | keine Verstellung |
| 30 - 32 | Zu niedrige Unterstützung | + 1 | keine Verstellung |
| 14 - 29 | Adäquate Unterstützung | keine Verstellung | keine Verstellung |
| 11 - 13 | Zu hohe Unterstützung | - 2 | keine Verstellung |
| 8 - 10 | Zu hohe Unterstützung | - 3 | keine Verstellung |
| < 8 | Bradypnoe | keine Verstellung | keine Verstellung |

### Bewertung des Tidalvolumens

| **Tidalvolumen/kg [ml/kg]** | **Diagnose** | **Δ PS [mbar [** | **PIF** |
|---|---|---|---|
| < 5 | Zu niedrige Unterstützung | + 2 | keine Verstellung |
| 5 - 10 | Adäquate Unterstützung | keine Verstellung | keine Verstellung |
| 11 - 12 | Zu hohe Unterstützung | - 2 | keine Verstellung |
| 13 - 14 | Zu hohe Unterstützung | - 3 | keine Verstellung |
| > 14 | Zu hohe Unterstützung | - 4 | keine Verstellung |

### Bewertung der Inspirationszeit

| **Inspirationszeit [s]** | **Diagnose** | **Δ PS [mbar]** | **PIF** |
|---|---|---|---|
| < 0.6 | Zu kurze Unterstützung | keine Verstellung | 25 |
| 0.6 - 1.2 | Adäquate Unterstützung | keine Verstellung | keine Verstellung |
| 1.3 - 1.7 | Zu lange Unterstützung | keine Verstellung | 50 |
| > 1.7 | Zu lange Unterstützung | keine Verstellung | 70 |

## Patentansprüche

1. Beatmungssystem zur nicht-invasiven Überdruckbeatmung,
- mit einer Druckquelle zur Bereitstellung von Atemgas mit steuerbarem Druck,
- mit einer Steuer- und Auswerteeinheit, die zum Steuern der Druckquelle ausgebildet ist und mit Sensoren verbunden ist, die die Erfassung des Leckagevolumens, der Spontanatemfrequenz, des Tidalvolumens und der Inspirationszeit ermöglichen, **dadurch gekennzeichnet, dass** die Steuer- und Auswerteeinheit dazu eingerichtet ist, automatisch folgende Messungen und Einstellungen in der aufgeführten Reihenfolge vorzunehmen:
i) das Leckagevolumen (MV_LEAK) daraufhin zu überprüfen, ob es oberhalb eines vorgegebenen Grenzwertes liegt, und wenn ja und wenn die Spontanatemfrequenz (F_SPON) innerhalb eines vorgegebenen Spontanatemfrequenzintervalls und das Tidalvolumen (VT) innerhalb eines vorgegebenen Tidalvolumenintervalls liegt, die inspiratorische Druckunterstützung zu reduzieren und wenn die Spontanatemfrequenz (F_SPON) außerhalb des vorgegebenen Spontanatemfrequenzintervalls oder das Tidalvolumen (VT) außerhalb des vorgegebenen Tidalvolumenintervalls liegt, einen Alarm auszulösen und zum Ausgangspunkt zurückzukehren und nur zu Schritt ii) überzugehen, wenn das Leckagevolumen unterhalb des vorgegebenen Grenzwertes liegt,
ii) wenn die Spontanatemfrequenz (F_SPON) oberhalb eines vorgegebenen Maximalwerts oder unterhalb eines vorgegebenen Minimalwerts liegt, einen Alarm auszulösen und zu Schritt i) zurückzukehren, und die Spontanatemfrequenz (F_SPON) mit dem vorgegebenen Spontanatemfrequenzintervall, das innerhalb des Intervalls zwischen Maximalwert und Minimalwert liegt, zu vergleichen, und falls die Spontanatemfrequenz (F_SPON) oberhalb des vorgegeben Spontanatemfrequenzintervalls liegt, die inspiratorische Druckunterschützung zu reduzieren, und falls die Spontanatemfrequenz (F_SPON) unter dem vorgegebenen Intervall liegt, die inspiratorische Druckunterschützung zu erhöhen und nach einer Erhöhung oder Reduzierung der inspiratorischen Druckunterschützung zu Schritt i) zurückzukehren, und falls die Spontanatemfrequenz (F_SPON) im vorgegebenen Spontanatemfrequenzintervall liegt, die inspiratorische Druckunterschützung unverändert zu lassen und zu Schritt iii) überzugehen,
iii) das Tidalvolumen (VT) daraufhin zu überprüfen, ob es innerhalb eines vorgegebenen Tidalvolumenintervalls liegt, und falls das Tidalvolumen oberhalb des vorgegeben Tidalvolumenintervalls liegt, die inspiratorische Druckunterstützung zu reduzieren, und falls das Tidalvolumen (VT) unter dem vorgegebenen Tidalvolumenintervall liegt, die inspiratorische Druckunterstützung zu erhöhen und nach Reduzierung oder Erhöhung zu Schritt i) zurückzukehren, und falls das Tidalvolumen (VT) im vorgegebenen Tidalvolumenintervall liegt, die inspiratorische Druckunterstützung unverändert zu lassen und zu Schritt iv) überzugehen,
iv) die Zeitperiode, in der die inspiratorische Druckunterstützung durchgeführt wird, in Abhängigkeit von der Inspirationszeit des Patienten einzustellen, indem die Zeitperiode der inspiratorischen Druckunterstützung erhöht wird, wenn die Inspirationszeit (TI_SPON) unter einem vorgegebenem Inspirationszeitintervall liegt, und sie verringert wird, wenn die Inspirationszeit (TI_SPON) über dem vorgegebenem Inspirationszeitintervall liegt, wobei sie unverändert belassen wird, wenn die Inspirationszeit im vorgegebenem Inspirationszeitintervall liegt, und zu Schritt i) zurückzukehren.

## Claims

1. A respiration system for non-invasive positive pressure respiration,
- having a pressure source for the provision of respiratory gas with controllable pressure,
- having a control and evaluation unit which is configured to control the pressure source and is connected to sensors which render possible the detection of the leakage volume, the spontaneous respiratory frequency, the tidal volume and the inspiration time, **characterised in that** the control and evaluation unit is set up to effect automatically successive measurements and settings in the sequence set out:
i) to check the leakage volume (MV_LEAK) to see whether it lies above a predetermined limiting value and if it does and if the spontaneous respiratory frequency (F_SPON) lies within a predetermined spontaneous respiratory frequency interval and the tidal volume (VT) lies within a predetermined tidal volume interval to reduce the inspiratory pressure support and if the spontaneous respiratory frequency (F_SPON) lies outside the predetermined spontaneous respiratory frequency interval or the tidal volume (VT) lies outside the predetermined tidal volume interval to trigger an alarm and to return to the starting point and only transition to step ii) if the leakage volume lies below the predetermined limiting value,
ii) if the spontaneous respiratory frequency (F_SPON) lies above a predetermined maximum value or below a predetermined minimum value to trigger an alarm and return to step i), and to compare the spontaneous respiratory frequency (F_SPON) with the predetermined spontaneous respiratory frequency interval that lies within the interval between the maximum value and the minimum value, and if the spontaneous respiratory frequency (F_SPON) lies above the predetermined spontaneous respiratory frequency interval to reduce the inspiratory pressure support and if the spontaneous respiratory frequency (F_SPON) lies below the predetermined interval to increase the inspiratory pressure support, and after an increase or reduction of the inspiratory pressure support to return to step i), and if the spontaneous respiratory frequency (F_SPON) lies in the predetermined spontaneous respiratory frequency interval to leave the inspiratory pressure support unchanged and to transition to step iii),
iii) to check the tidal volume (VT) to see whether it lies within a predetermined tidal volume interval and if the tidal volume lies above the predetermined tidal volume interval to reduce the inspiratory pressure support, and if the tidal volume (VT) lies below the predetermined tidal volume interval to increase the inspiratory pressure support, and after a reduction or increase to return to step i), and if the tidal volume (VT) lies in the predetermined tidal volume interval to leave the inspiratory pressure support unchanged and to transition to step iv),
iv) to set the time period in which the inspiratory pressure support is carried out as a function of the.inspiration time of the patient **in that** the time period of the inspiratory pressure support is increased if the inspiration time (TI_SPON) lies below a predetermined inspiration time interval and it is decreased if the inspiration time (TI_SPON) lies above the predetermined inspiration time interval, wherein it is left unchanged if the inspiration time lies in the predetermined inspiration time interval, and to return to step i).

## Revendications

1. Système de ventilation dévolu à la ventilation non invasive par pression positive, comprenant
- une source de pression, conçue pour fournir un gaz respiratoire dont la pression peut être commandée,
- une unité de commande et d'évaluation, réalisée en vue de commander ladite source de pression et raccordée à des capteurs permettant la détection du volume de fuite, de la fréquence respiratoire spontanée, du volume courant et du temps d'inspiration, **caractérisé par le fait que** l'unité de commande et d'évaluation est agencée pour effectuer automatiquement, dans l'ordre de succession indiqué, les mesures et réglages suivants :
i) vérifier le volume de fuite (MV_LEAK) pour déterminer s'il est supérieur à une valeur limite préétablie et diminuer l'aide inspiratoire dans l'affirmative, et lorsque la fréquence respiratoire spontanée (F_SPON) se situe à l'intérieur d'un intervalle préétabli de fréquence respiratoire spontanée et lorsque le volume courant (VT) se situe à l'intérieur d'un intervalle préétabli de volume courant ; et, lorsque ladite fréquence respiratoire spontanée (F_SPON) se situe en dehors dudit intervalle préétabli de fréquence respiratoire spontanée ou lorsque ledit volume courant (VT) se situe en dehors dudit intervalle préétabli de volume courant, déclencher une alarme et retourner au point initial, et passer à l'étape ii) uniquement lorsque ledit volume de fuite est inférieur à ladite valeur limite préétablie,
ii) lorsque ladite fréquence respiratoire spontanée (F_SPON) est supérieure à une valeur maximale préétablie ou inférieure à une valeur minimale préétablie, déclencher une alarme et retourner à l'étape i), et comparer ladite fréquence respiratoire spontanée (F_SPON) audit intervalle préétabli de fréquence respiratoire spontanée qui se trouve à l'intérieur de l'intervalle compris entre ladite valeur maximale et ladite valeur minimale ; et diminuer l'aide inspiratoire dans le cas où ladite fréquence respiratoire spontanée (F_SPON) se situe au-delà dudit intervalle préétabli de fréquence respiratoire spontanée ; et, dans le cas où ladite fréquence respiratoire spontanée (F_SPON) se situe en deçà dudit intervalle préétabli, augmenter l'aide inspiratoire et retourner à l'étape i) après une augmentation ou une diminution de ladite aide inspiratoire ; et, dans le cas où ladite fréquence respiratoire spontanée (F_SPON) se situe dans ledit intervalle préétabli de fréquence respiratoire spontanée, laisser l'aide inspiratoire inchangée et passer à l'étape iii),
iii) vérifier le volume courant (VT) pour déterminer s'il se situe à l'intérieur d'un intervalle préétabli de volume courant, et diminuer l'aide inspiratoire dans le cas où ledit volume courant se situe au-delà dudit intervalle préétabli de volume courant ; et, dans le cas où ledit volume courant (VT) se situe en deçà dudit intervalle préétabli de volume courant, augmenter l'aide inspiratoire et retourner à l'étape i) après diminution ou augmentation ; et, dans le cas où ledit volume courant (VT) se situe dans ledit intervalle préétabli de volume courant, laisser l'aide inspiratoire inchangée et passer à l'étape iv),
iv) régler, en fonction du temps d'inspiration du patient, la période durant laquelle l'aide inspiratoire est exécutée, en accroissant ladite période d'aide inspiratoire lorsque ledit temps d'inspiration (TI_SPON) se situe en deçà d'un intervalle préétabli de temps d'inspiration, et en la réduisant lorsque ledit temps d'inspiration (TI_SPON) se situe au-delà dudit intervalle préétabli de temps d'inspiration, ladite période étant laissée inchangée lorsque ledit temps d'inspiration se situe dans ledit intervalle préétabli de temps d'inspiration, et retourner à l'étape i).
